# EUROPEAN PATENT APPLICATION

(11) **EP 0 637 434 A1**
(43) Date of publication of application: **08.02.1995**
(21) Application number: 94304957.7
(22) Date of filing: 05.07.1994
(51) Int. Cl.: A61B 17/17

(54) **Drill guide for external fixation devices**

(30) Priority: 08.07.1993 US 88988
(71) Applicant: SMITH & NEPHEW RICHARDS, INC., Memphis, Tennessee 38116 (US)
(72) Inventor: Gosney, Mark S., Memphis, Tennessee 38119 (US); Crosslin, Marietta I., Horn Lake, MS 38637 (US); Dominquez, Leonel, Germantown, Tennessee 38138 (US); Herzenberg, John E., Owings Mills, Maryland 21117 (US)
(74) Representative: Gilholm, Stephen Philip

(57) **Abstract**

A drill guide (10) and method for guiding placement of external fixation pins in the bone (B) of a human limb to avoid an implanted intramedullary nail (14) with the drill guide means having a plurality of guide holes (24) adapted to guide and receive a bone drill bit and bone pins (18). The drill guide means also includes connecting means for connecting the drill guide means to one end of a drill guide handle (12) connected to an intramedullary nail implanted in the bone and for aligning the guide holes in a fixed position relative to the nail so that the hole centerlines are oriented to avoid the nail.

## Description

The present invention relates to drill guides used in orthopedic surgery, and more particularly to a drill guide and method for guiding the placement of external fixation pins in the bone of a human limb to avoid an implanted intramedullary nail.

Limb lengthening and bone transport are often performed with external fixation devices that fix separate bone segments and then displace the bone segments axially. Bone lengthening procedures are performed with a variety of external fixation devices and the success of these procedures is influenced by the mechanical stability of the lengthening system used.

When an intramedullary nail is used in conjunction with the external fixation device the stability of the lengthening system is greatly increased and the success of the lengthening system is enhanced. The external fixation frame is applied after the nail is implanted in the intramedullary canal of the bone being lengthened. The external frame is connected to the bone with various pins and wires and in connecting the frame to the bone it is necessary to place the pins and wires in the bone allowing for adequate bone purchase but without coming in contact with the implanted nail. Presently, the only way to miss the implanted intramedullary nail, when placing the fixation pins and wires in the bone, is by free-handing the insertion of the pins and wires with image intensification.

Drill guides currently available are for guiding bone screws through implanted intramedullary nails for securing the nail in the intramedullary canal of a bone. These drill guides are connected to the implanted intramedullary nail and aid the surgeon in aligning the drill bit with the preformed holes in the intramedullary nail.

With many intramedullary nail drill guides, a handle or some other device is aligned and securely fastened to the exposed proximal end of the implanted intramedullary nail. A second section of the drill guide has guide holes that align with the screw holes in the implanted nail when the guide is placed in proper alignment on the handle. The orientation of the drill guide insures that the holes drilled into the bone will align with the holes in the intramedullary nail for the proper placement of the bone screws.

It would be advantageous to have a radiolucent or radiotransparent drill guide for use in the placement of pins and wires connecting an external fixation frame to a bone that avoids an implanted intramedullary nail. It would also be advantageous to have a drill guide for the placement of bone pins and wires that could be used with any intramedullary nail and external fixation system that allows adequate bone purchase while avoiding the implanted intramedullary nail.

According to the invention we provide a drill guide for guiding placement of external fixation pins in the bone of a human limb while avoiding an implanted intramedullary nail, comprising:
a) a drill guide means for guiding a drill bit so that external fixator pin holes can be formed in the bone of a human limb, the drill guide means including a drill guide body with a plurality of guide holes therethrough, each adapted to receive a drill bit and bone pins;
b) the drill guide means including connecting means for connecting the drill guide means to the intramedullary nail implanted in the bone while aligning the guide holes in a fixed position relative to the nail so that the hole centerlines extend along a side of the nail, enabling a drill to track a selected opening during drilling, and avoid the nail.

The invention is directed to a novel radiolucent or radiotransparent drill guide for guiding the placement of external fixation pins in the bone of a human limb while avoiding an implanted intramedullary nail. The drill guide is to be used in conjunction with a proximal drill guide for an intramedullary nail and is generally elongated in shape with a plurality of guide holes adapted to receive a bone drill bit and bone pins. The drill guide has a slotted keyed opening for connecting to the proximal drill guide handle connected to the implanted intramedullary nail. The keyed opening of the drill guide fits into a keyway on the proximal drill guide handle in order to align the guide holes of the drill guide in a fixed position relative to the nail so that the guide hole centerlines are oriented to avoid the implanted nail.

In a preferred embodiment, the guide holes are positioned in two parallel lines on the longitudinal axis of the drill guide and are dimensioned to allow for the placement of a drill sleeve through the guide holes. The guide holes are placed on the drill guide such that the guide can be used on the left or right side of a human body and can be used with any intramedullary nail and external fixation system.

We further provide a drill guide for guiding placement of external fixation pins in the bone of a human limb while avoiding an implanted intramedullary nail, comprising:
a) a drill guide means for guiding a drill bit so that external fixator pin holes can be formed in the bone of a human limb, the drill guide means including a drill guide body with a plurality of guide holes therethrough, each adapted to receive a drill bit and bone pins, the drill guide means having a central longitudinal axis;
b) the drill guide means including connecting means for connecting the drill guide means to the implanted intramedullary nail having a central longitudinal axis, while aligning the guide holes in a fixed position relative to the nail so that the hole centerlines extend along a side of the nail, enabling a drill to track a selected opening during drilling, and avoid the nail;
c) the connecting means including a proximal drill guide handle having a free end portion, an attachment portion, and a central longitudinal axis, the attachment portion including connecting means for connecting the drill guide handle to the implanted nail;
d) the central longitudinal axis of the drill guide handle forming a first right angle with the axis of the implanted intramedullary nail and the central longitudinal axis of handle forming a second right angle with the central longitudinal axis of the drill guide means when the drill guide means is connected to the free end of the drill guide handle so as to provide parallel alignment of the central axis of the intramedullary nail with the central axis of the drill guide means.

The present invention also includes a method for guiding the placement of the external fixation pins in the bone of a human while avoiding the implanted intramedullary nail.

According to a further feature of the invention we provide a method for guiding the placement of external fixation pins in the bone of a human limb while avoiding an implanted intramedullary nail, comprising the steps of:
a) connecting a drill guide means to the intramedullary nail implanted in the bone;
b) positioning the drill guide means relative to the implanted intramedullary nail such that at least a plurality of the guide hole centerlines extend along a side of the nail, enabling a bone drill and bone pins to be oriented to avoid the nail;
c) placing a bone drill bit through selected guide holes such that holes can be formed in the bone of the human limb for receiving fixation pins in the bone;
d) drilling holes in the bone for receiving one or more fixation pins;
e) inserting one or more fixation pins, for supporting an external fixation device, into the bone of a human limb.

We also provide a kit for guiding placement of external fixation pins in the bone of a human limb while avoiding an implanted intramedullary nail, comprising:
a) a plurality of guide blocks defining a different configuration of spacing of guide holes, each different configuration of guide holes adapted to receive bone pins of different fixation devices;
b) a common handle adapted to receive the plurality of guide blocks.

A better understanding of the invention can be obtained when the detailed description of exemplary embodiments set forth below is viewed in conjunction with the accompanying drawings, in which:
FIGURE 1 is a perspective view of the present invention in use on the bone of a human limb;
FIGURE 2 is an exploded side view of the present invention attached to the handle of a proximal drill guide;
FIGURE 3 is a partial cutaway front elevation view of the present invention;
FIGURE 4 is a partial cutaway side elevation view of the invention of Fig. 3;
FIGURE 5 is a top partially sectioned view of the invention of Fig. 1 illustrating a drill placed through a drill sleeve;
FIGURE 6 is a horizontal sectional view of the invention illustrating non-angled guide holes;
FIGURE 7 is a horizontal sectional view of the invention illustrating angled guide holes.

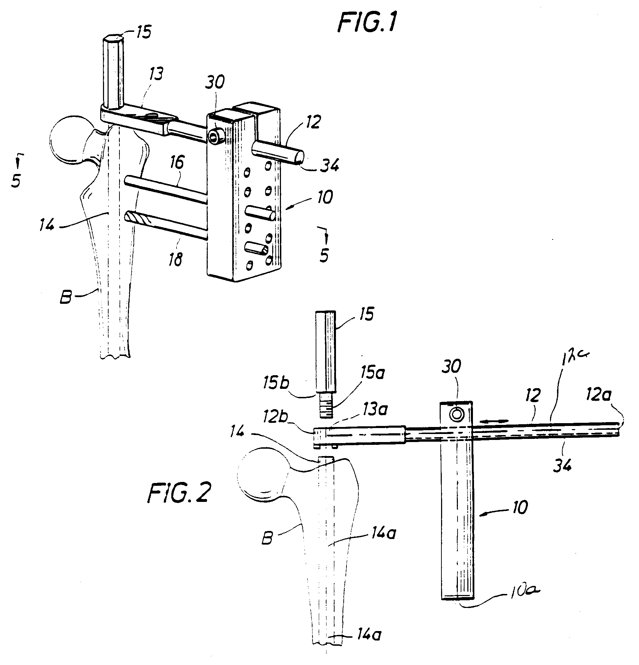

The present invention is directed to a drill guide to be used in conjunction with a proximal drill guide for an intramedullary nail. In Fig. 1, a patient's femur is designated as bone B, which has an intramedullary canal that receives an intramedullary nail 14 of any diameter, for example from 8mm to 15mm.

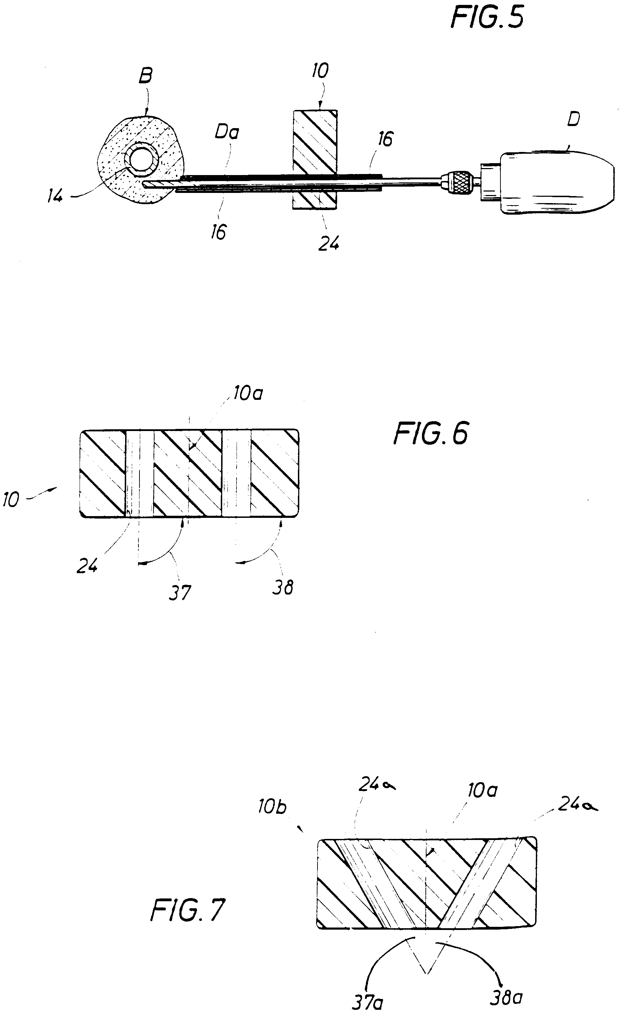

Drill guide 10 of the present invention is used for guiding the placement of bone pins, self-drilling pins, bone taps and wires used with external fixation devices when an implanted intramedullary nail 14 is part of a limb lengthening system. The drill guide 10 can place bone pins and wires anywhere on the bone B, but preferably the pins and wires are placed as close as possible to the nail 14, for example within 1 mm of the nail, for maximum bone purchase (Fig. 5). The drill guide 10, with a plurality of guide holes 24, is placed on a proximal drill guide handle 12 that is connected to the implanted intramedullary nail 14 with a bolt 15. The drill guide 10 and the handle 12 could also be constructed as a single unit with the handle portion being connected to the implanted intramedullary nail 14 with the bolt 15.

If desired, a drill sleeve 16 can be placed through a selected one of the guide holes 24 for guiding a bone drill D directly to the bone B. The guide holes 24 provide the proper placement of the holes in the bone B of a human limb for receiving the bone pins 18 used in an external fixation system.

The proximal drill guide handle 12 provides a free end portion 12a and an attachment end portion 12b. The attachment end portion 12b includes enlarged portion 13 having a cylindrically shaped opening 13a. The opening 13a provides an axis that coincides with the central axis 14a of the nail 14 upon assembly which forms a right angle with the central longitudinal axis 12c of the handle 12 (Figs. 1 and 2).

Bolt 15 provides a lower, externally threaded cylindrical portion 15a that communicates with annular shoulder 15b. The upper proximal end portion of nail 14 provides internal threads that engage the externally threaded portion 15a of bolt 15 during use. The opening 13a is generally cylindrically shaped and slightly larger than the threaded portion 15a of the bolt 15. The externally threaded portion 15a extends through the opening 13a and engages internal threads at the top of the implanted nail 14.

Bolt 15 can provide a hexagonal outer surface as shown in Fig. 1. A surgeon can grip and apply torque to the bolt 15 for firmly affixing the bolt 15 to the top of the nail 14, thereby rigidifying the assembly of bolt 15, drill guide handle 12, and nail 14. In this position, the central longitudinal axis 12c of the drill guide handle 12 forms a right angle with the axis 14a of the nail 14 with the axis 14a being essentially linear at the proximal portion of the nail 14. Drill guide 10 provides a central axis 10a that forms a right angle with the central longitudinal axis 12c of drill guide handle 12 upon assembly. A known geometric relationship between the drill guide 10, drill guide handle 12, and nail 14 is formed upon assembly. Figs. 1 and 2 illustrate the assembly of the drill guide 10 upon the drill guide handle 12.

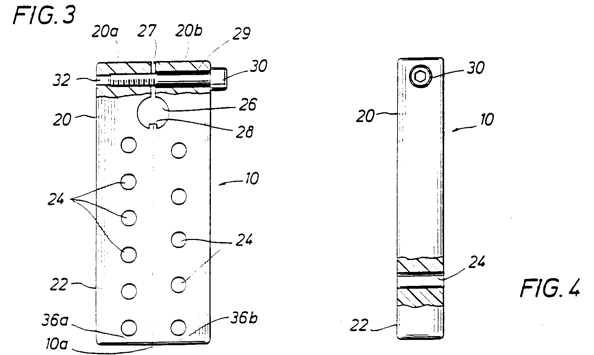

In Figs. 1, 3 and 4, drill guide 10 can be generally in the shape of an elongated block of material, measuring for example approximately 5 inches high, 2 inches across and 3/4 inches wide. In a preferred embodiment, the drill guide 10 is formed of a radiolucent or radiotransparent material.

Drill guide 10 has a proximal end 20 and a distal end 22. Proximal end 20 has a pair of opposed portions 20a, 20b separated by opening 26 and slotted portion 27. The proximal end 20 contains a generally cylindrically shaped opening 26 adapted to receive drill guide handle 12. Ridge or key portion 28 is positioned at opening 26 to cooperate with a slot or keyway 34 located on the underside of the handle 12, as illustrated in Figs. 1 and 2.

An internally threaded opening 32 is positioned on the transverse axis of the portion 20a and a sleeve 29 extends through portion 20b of proximal end 20 of the drill guide 10. Tightening screw 30 threads through the sleeve 29 and engages the internally threaded opening 32, in order to clamp the handle 12 on the drill guide 10.

In a preferred embodiment, the plurality of guide holes 24 are oriented on the longitudinal axis of the drill guide 10 in two parallel rows 36a,b. The two rows 36a,b are positioned on either side of the drill guide centerline 10a. The guide holes 24 can be positioned on the drill guide 10 a accommodate a variety of fixation devices and a kit is provided that contains a plurality of drill guides, for example drill guide 10, 10b, for use with a common handle 12. Each drill guide has a different guide hole configuration for accommodating a different fixation device.

The keyway 34 on the handle 12 aligns with the centerline 14a of the nail 14. When the opening 26 of the drill guide 10 is properly placed on the keyway 34, the centerline 14a of the nail 14 is in parallel alignment with the centerline 10a of the drill guide 10. The guide holes 24 are offset dimensionally from the parallel nail and drill guide centerlines 14a, 10a such that the guide holes 24 will place the pins 18 on the bone B so as to avoid the implanted intramedullary nail 14 (Fig. 5). It is necessary for the drill guide 10 to be positioned on the handle 12 such that the axis of the guide holes 24 are correctly positioned to avoid the nail 14. This correct position may include angling the guide holes 24 or the drill guide 10 to allow for a greater bone purchase of the bone pins 18.

Additionally, the guide holes 24 are positioned on the drill guide 10 in such a way as to allow use of the drill guide 10 on either the left or right side of a human body. The guide holes 24 can be located to accommodate the dimensions of any intramedullary nail and external fixation device as part of a bone lengthening system.

Drill sleeves, which are known in the art, can also be used with the drill guide 10. As shown in Fig. 1 and 5, a drill sleeve 16 can be placed through the guide hole 24 making contact with the bone B to prevent a drill bit Da of a bone drill D, placed through the drill sleeve 16, from walking or skiving on the bone B. The drill guide 10 can be used at any distance from the bone B if pins 18 are to be placed at right angles 37, 38 to the axis of the nail centerline 14a as illustrated in Fig. 6. However, to reduce drill point walking, a drill guide 10b with angled guide holes 24 can be used. As illustrated in Fig. 7, guide holes 24a can be angled towards the nail centerline 14a, at the angle 37a, 38a, in order to approach the surface of bone B more directly with the drill bit Da. The angle 37a, 38a can be in the range of 0 to 50° and angled guide holes 24a require that the drill guide 10b be placed at a fixed distance from the nail 14.

The drill guide is intended to guide pin and wire placement about the proximal or driving portion of an intramedullary nail as the best pin placement anatomically in the femur is generally posterior to the intramedullary canal. However, anterior pin placement is possible, especially at higher angles if using an extension device.

In the placement of the fixation pins, the drill guide 10 is used in conjunction with a proximal drill guide for an intramedullary nail or the drill guide 10 and handle 12 can be formed as a single unit. The drill guide 10 is connected to the proximal drill guide handle 12 that is connected to an implanted intramedullary nail 14. The drill guide 10 is positioned on the handle 12 such that the keyed opening 26 of the drill guide 10 engages the keyway 34 on the handle 12. The drill guide 10 is secured to the handle 12 by tightening the screw 30. A bone drill D is placed through the guide holes 24 and holes are drilled in the bone as close as possible to the nail 14 for maximum bone purchase of the pins 18 without hitting the implanted nail 14 (Fig. 5). The fixation pins 18 are then inserted into the bone B for securing an external fixation device onto the bone B of a human limb.

The radiolucent or radiotransparent drill guide of the present invention allows the placement of pins and wires connecting an external fixation frame to a bone while avoiding an implanted intramedullary nail. The drill guide and method of the present invention can be used with any intramedullary nail and external fixation device used in a bone lengthening system.

It should be understood that there can be improvements and modifications made to the embodiments of the invention described in detail above without departing form the spirit or scope of the invention, as set forth in the accompanying claims.

## Claims

1. A drill guide for guiding placement of external fixation pins in the bone of a human limb while avoiding an implanted intramedullary nail, comprising:
a) a drill guide means for guiding a drill bit so that external fixator pin holes can be formed in the bone of a human limb, the drill guide means including a drill guide body with a plurality of guide holes therethrough, each adapted to receive a drill bit and bone pins;
b) the drill guide means including connecting means for connecting the drill guide means to the intramedullary nail implanted in the bone while aligning the guide holes in a fixed position relative to the nail so that the hole centerlines extend along a side of the nail, enabling a drill to track a selected opening during drilling, and avoid the nail.

2. The drill guide of claim 1, wherein the drill guide means is elongated in shape with the plurality of guide holes oriented in two parallel rows positioned on the longitudinal axis of the drill guide means.

3. The drill guide of claim 1, wherein the connecting means includes a slotted keyed hole for connecting and aligning the drill guide means on the drill guide handle connected to the implanted intramedullary nail.

4. The drill guide of claim 3, wherein the keyed hole cooperates with a keyway on the drill guide handle connected to the implanted intramedullary nail for aligning the drill guide means with the implanted nail.

5. The drill guide of claim 3, wherein the connecting means includes a screw means for tightening the slotted keyed hole of the drill guide means to the drill guide handle connected to the implanted intramedullary nail.

6. The drill guide of claim 1, wherein the guide means is formed of a radiolucent or radiotransparent material.

7. The drill guide of claim 1, wherein the plurality of guide holes are positioned on the drill guide means to allow use on the left or right side of a human body.

8. The drill guide of claim 2, wherein the plurality of holes are angled in relation to the longitudinal axis of the drill guide means, the angle being in the range of 0 to 50°.

9. The drill guide of claim 1, wherein the plurality of guide holes are dimensioned to allow for the placement of a drill sleeve through the openings to make contact with the bone of a human.

10. The drill guide of claim 1, wherein the guide means can attach to the intramedullary nail drill guide handle at any distance from a human bone having an implanted intramedullary nail.

11. A drill guide for guiding placement of external fixation pins in the bone of a human limb while avoiding an implanted intramedullary nail, comprising:
a) a drill guide means for guiding a drill bit so that external fixator pin holes can be formed in the bone of a human limb, the drill guide means including a drill guide body with a plurality of guide holes therethrough, each adapted to receive a drill bit and bone pins, the drill guide means having a central longitudinal axis;
b) the drill guide means including connecting means for connecting the drill guide means to the implanted intramedullary nail having a central longitudinal axis, while aligning the guide holes in a fixed position relative to the nail so that the hole centerlines extend along a side of the nail, enabling a drill to track a selected opening during drilling, and avoid the nail;
c) the connecting means including a proximal drill guide handle having a free end portion, an attachment portion, and a central longitudinal axis, the attachment portion including connecting means for connecting the drill guide handle to the implanted nail;
d) the central longitudinal axis of the drill guide handle forming a first right angle with the axis of the implanted intramedullary nail and the central longitudinal axis of handle forming a second right angle with the central longitudinal axis of the drill guide means when the drill guide means is connected to the free end of the drill guide handle so as to provide parallel alignment of the central axis of the intramedullary nail with the central axis of the drill guide means.

12. The drill guide of claim 11, wherein the drill guide handle and drill guide means are separate and are removable relative to each other.

13. The drill guide of claim 11, wherein the drill guide means is elongated in shape with the plurality of guide holes oriented in two parallel rows positioned on the longitudinal axis of the drill guide means.

14. The drill guide of claim 11, wherein the connecting means includes a slotted keyed hole for connecting and aligning the drill guide means on the drill guide handle connected to the implanted intramedullary nail.

15. The drill guide of claim 14, wherein the keyed hole cooperates with a keyway on the drill guide handle connected to the implanted intramedullary nail for aligning the drill guide means with the implanted nail.

16. The drill guide of claim 14, wherein the connecting means includes a screw means for tightening the slotted keyed hole of the drill guide means to the drill guide handle connected to the implanted intramedullary nail.

17. The drill guide of claim 11, wherein the guide means is formed of a radiolucent or radiotransparent material.

18. A kit for guiding placement of external fixation pins in the bone of a human limb while avoiding an implanted intramedullary nail, comprising:
a) a plurality of guide blocks defining a different configuration of spacing of guide holes, each different configuration of guide holes adapted to receive bone pins of different fixation devices;
b) a common handle adapted to receive the plurality of guide blocks.
